# EUROPÄISCHE PATENTSCHRIFT

(11) **EP 1 926 456 B1**
(45) Veröffentlichungstag und Bekanntmachung des Hinweises auf die Patenterteilung: **16.11.2011**
(21) Anmeldenummer: 06791566.0
(22) Anmeldetag: 08.08.2006
(51) Int. Cl.: A61F 2/32

(54) **BLATTARTIGER SCHAFT EINER HÜFTGELENKPROTHESE**
BLADE-LIKE SHAFT OF A HIP-JOINT PROSTHESIS
TIGE EN FORME DE LAME D'UNE PROTHESE DE HANCHE

(30) Priorität: 20.09.2005 DE 102005044872; 12.10.2005 DE 102005048873
(43) Veröffentlichungstag der Anmeldung: 04.06.2008
(73) Patentinhaber: Smith & Nephew Orthopaedics AG, 6343 Rotkreuz (CH)
(72) Erfinder: MOSER, Walter, 5015 Erlinsbach (CH); SEIDL, Alex, 8044 Zürich (CH); WUNDERLE, Dirk, 8037 Zürich (CH)
(74) Vertreter: Popp, Eugen
(86) Internationale Anmeldenummer: PCT/EP2006/007842
(87) Internationale Veröffentlichungsnummer: WO 2007/033727

(56) Entgegenhaltungen:
- EP-A2- 0 032 165
- EP-A2- 0 145 939
- FR-A- 2 678 510

## Beschreibung

Die Erfindung betrifft einen blattartigen Schaft einer Hüftgelenkprothese für die Verankerung im Femur, mit einem einen Prothesenhals umfassenden Abschnitt einerseits und einem sich zu einem distalen Ende hin verjüngenden Femur-Verankerungsabschnitt andererseits.

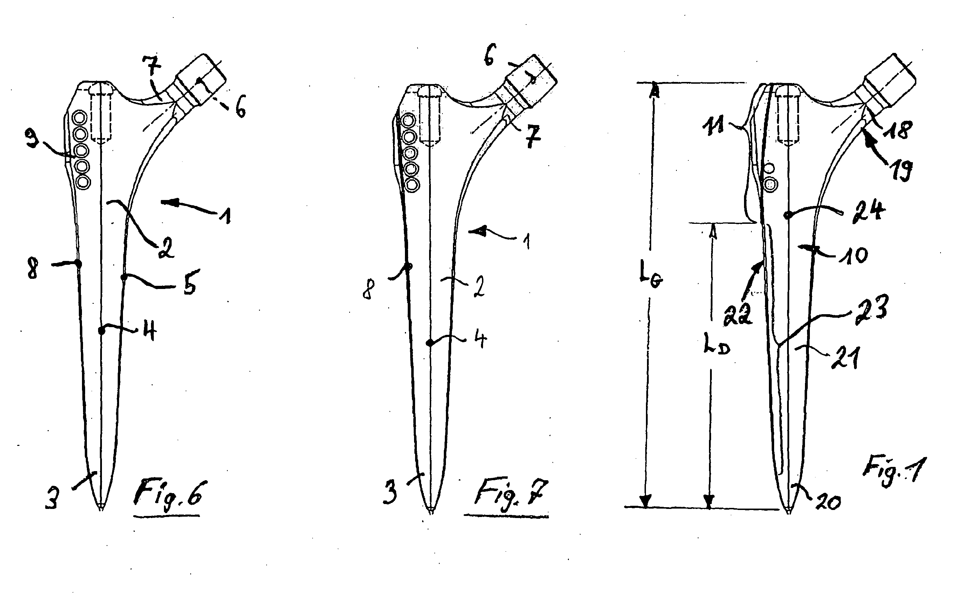

Derartige blattartige Schäfte sind allgemein bekannt. Es wird diesbezüglich nur beispielhaft auf die EP 0 240 815 B1 verwiesen. Dort ist ein Schaft entsprechend Fig. 6 dargestellt und beschrieben. Dementsprechend umfaßt dieser Schaft 1 einen einen Prothesenhals 7 umfassenden Abschnitt einerseits und einen sich zu einem distalen Ende 3 hin verjüngenden Femur-Verankerungsabschnitt 2 andererseits. Dieser Femur-Verankerungsabschnitt erweitert sich vom distalen Ende 3 in Richtung der Schaft-Längsachse 4 allseitig konisch. Die mediale Schmalseite 5 geht aus dem erwähnten Konus heraus in einen stetig gekrümmten Bogen über, der in einer Ebene endet, die senkrecht zur Prothesenhalsachse 6 verlaufend den Prothesenhals 7 gegen das Schaftblatt bzw. den Femur-Verankerungsabschnitt 2 abschließt. Der Prothesenhals 7 endet in einem sich nach außen konisch verjüngenden Zapfen, auf den ein nicht gezeigter kugelförmiger Gelenkkopf aufsteckbar ist.

Die laterale Schmalseite 8 ist aus der konischen Erweiterung heraus zu einem Trochanterflügel 9 ausgeweitet, ehe sie über eine Schulter des Schaftblatts bzw. Verankerungsabschnitts in die erwähnte Prothesenhals-Abschlußebene einmündet.

Der beschriebene Schaft dient zur unzementierten Verankerung im Femur. Grundsätzlich soll sich die vorliegende Erfindung jedoch auch auf zementierte Schäfte beziehen.

In beiden Fällen muß im Femur vorab ein Schaft-Aufnahmeraum bzw. eine entsprechende Kavität geschaffen werden, und zwar mittels eines der Form des Schaftes entsprechenden Forminstrumentes, insbesondere Raspel. Diese Forminstrumente bzw. Raspeln entsprechen exakt der Geometrie des jeweiligen Schaftes, oder weichen gezielt davon ab, um ein vorbestimmtes Untermaß für einen press-fit oder ein vorbestimmtes Übermaß als Raum für einen Zementmantel zu erhalten.

Nach Öffnung des Hüftgelenks und Resektion des Schenkelhalses wird im proximalen Femur das knöcherne Lager zur Aufnahme des Verankerungsschaftes präpariert. Entsprechend der Schaftform wird das knöcherne Verankerungsbett mit einem geeigneten Forminstrument, insbesondere Raspel, durch Absenken entlang der Schaftachse erzeugt. Zum Absenken in den mit spongiösem Knochen und Weichgewebe gefüllten Markraum wird die Raspel über ein als Hammer wirkendes Gewicht oder mit einem anderen geeigneten Instrument vorwärts getrieben. Im Falle einer gekrümmten Schaftachse wird das Forminstrument bzw. die Raspel längs einer gekrümmten Bahn bogenförmig abgesenkt, während bei einer geraden Schaftachse die Raspel längs einer Geraden, welche im wesentlichen der Achse des proximalen Markraums entspricht, vorangetrieben wird.

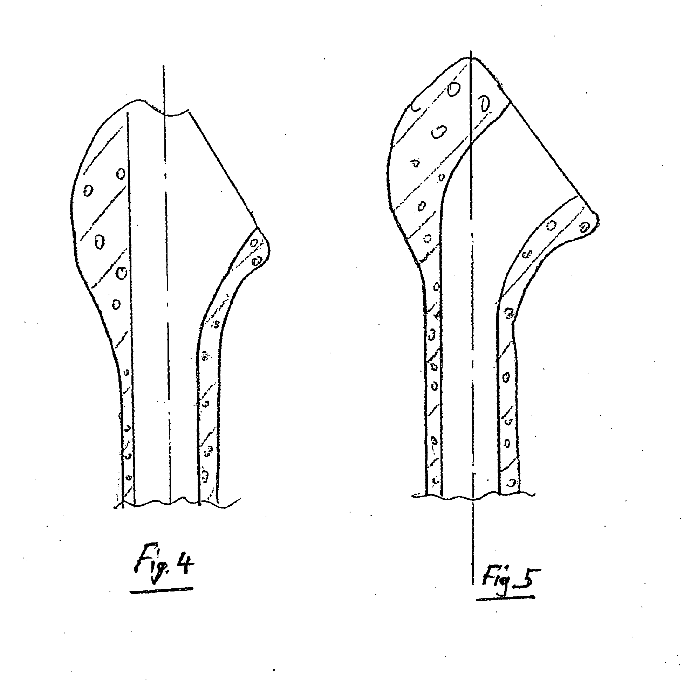

Für eine unzementierte Verankerung von Hüftschäften hat sich die Ausführung des Verankerungsabschnitts als Geradschaft klinisch besonders bewährt. Es wird diesbezüglich auch auf die bekannten Schäfte gemäß der EP 0 145 939 A2 und EP 0 032 165 A2 hingewiesen, die sich allesamt dadurch auszeichnen, daß der Geradabschnitt sich über eine Länge von etwa 60% bis 75% der Gesamtlänge des Schaftes erstreckt. Sichere Implantationstechnik, eine hohe Primärstabilität und ein gutes Einwachsverhalten zeichnen dieses Konzept aus. Die Operationstechnik dieser Schäfte bedingt jedoch, daß der Markraum nicht nur in der Ebene der Resektionsfläche des Schenkelhalses, sondern weiter nach lateral in den Bereich des großen Trochanters geöffnet wird. Es wird diesbezüglich auf Fig. 4 verwiesen. Diese Figur zeigt, daß auch eine Resektion von Anteilen der Sehnenansätze in diesem Bereich notwendig ist. Das Ausmaß dieser Resektion hängt natürlich von der individuellen Form des proximalen Femurs und von der Formgebung des Geradschafts ab.

In jüngerer Zeit werden Implantationen von Gelenkendoprothesen zunehmend mit minimalinvasiven Operationstechniken vorgenommen. Ziel dieser Techniken ist eine schnellere Rehabilitation des Patienten, die mit geringerer Schmerzbelastung und kürzerem Krankenhausaufenthalt verbunden ist. Bei minimalinvasiven Operationstechniken wird das Operationstrauma, insbesondere bezüglich der funktional maßgebenden Strukturen gering gehalten. Für die Funktion des Hüftgelenks sind die Muskeln und Sehnen wesentliche Strukturmerkmale. Ziel der minimalinvasiven Implantationstechniken ist unter anderem die Vermeidung von Resektionen sowie Ablösungen von Sehnen- und Muskelansätzen im Bereich des großen Trochanters. Klassische Geradschäfte haben somit Nachteile für die Anwendung minimalinvasiver Techniken.

Zur Vermeidung von Resektionen im Bereich der Sehnenansätze am großen Trochanter kann bei Geradschäften die laterale Partie im Bereich des Trochanters abgeschrägt werden. Geradschäfte mit abgeflachter Schulterpartie sind grundsätzlich bekannt. Es wird diesbezüglich nur beispielhaft auf den sogenannten Müller-Geradschaft hingewiesen, der in "Technique d'implantation de prothèses totales de Müller par voie latérale transglutéale", Encyclopédie Medico-Chirurgicale (Paris) 44666, 1991, dargestellt und beschrieben ist.

Ziel dieser abgeflachten Schulter ist die Vermeidung größerer Defekte im Bereich des Trochantermassivs. Bei der Gestaltung dieser Abflachung wurde in der Regel ein im Größensystem konstanter Anteil der lateralen Schaftpartie mit einem Geradzug schräg zur Schaftachse und mit einem Radius gestaltet. Die zum Implantat korrespondierende Raspel wurde meist geometriegleich zum Implantat gestaltet. Technisch betrachtet wird mit der Raspel eine Hinterschneidung im Bereich des großen Trochanters erzeugt, so wie dies Fig. 5 erkennen läßt.

Bei der Implantation eines Hüftschaftes wird mit Raspeln aufsteigender Größe das knöcherne Bett bis zu der am besten passenden Größe geformt. Die jeweilige Raspel folgt dabei der Form des bereits vorhandenen Betts, gestaltet durch die vorhergehende Raspelgröße. Da der distale Anteil des Geradschaftes durch Absenken entlang einer geraden Achse erfolgt, ergeben sich für die geneigte oder gekrümmte Schulterpartie Kompromisse bei der Paßgenauigkeit. Diese wird jedoch durch die Raspeltechnik des Operateurs und die individuelle Knochenqualität beeinflußt.

Ausgehend vom genannten Stand der Technik ist es Aufgabe der vorliegenden Erfindung, einen blattartigen Schaft der eingangs genannten Art zu schaffen, der sich besonders gut für minimalinvasive Operationstechniken eignet. Dabei sollen die Vorteile herkömmlicher Geradschaftimplantate erhalten bleiben. Muskel- und Sehnenansätze sollen jedoch möglichst geschont werden.

Diese Aufgabe wird erfindungsgemäß durch die kennzeichnenden Merkmale des Anspruches 1 gelöst.

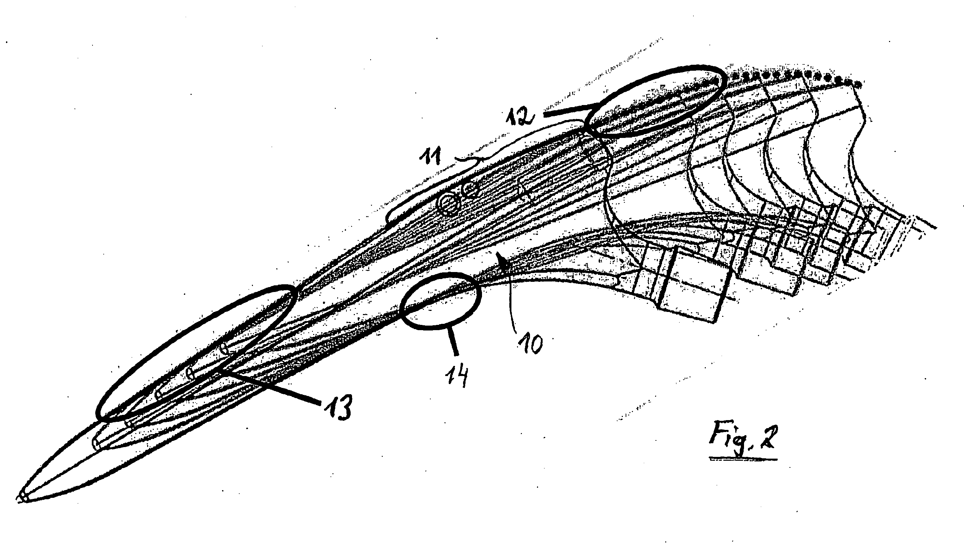

Besonders bedeutend ist der Verlauf des konvexen proximalen Bogenabschnitts, wobei sich dieser dadurch auszeichnet, daß der Bogenabschnitt als "Schleppkurve" ausgebildet ist, die das proximale Ende der lateralen Schmalseite des Schaftes bei Einführung desselben (oder einer entsprechenden Raspel) in eine komplementäre Kavität im Femur unter Aufrechterhaltung des Kontaktes zwischen lateral-distaler und proximal-medialer Schaftkontur einerseits und zugeordneter Begrenzung der Kavität andererseits beschreibt bzw. definiert. Die optimale Form der proximal-lateralen Schulterpartie ergibt sich also aus der Führung des Schaftes im knöchernen Bett, so wie dies in Fig. 2 dargestellt ist. Die lateral-proximale Schaftrundung 11 des Schaftes 10 entspricht der Kurve 12, die das proximale Ende der lateralen Schmalseite des Schaftes 10 bei Einführung desselben in eine komplementäre Kavität im Femur beschreibt unter der Bedingung, daß der Kontakt zwischen lateral-distaler Schaftkontur 13 und proximal-medialer Schaftkontur 14 einerseits und zugeordneter Begrenzung der hier nicht dargestellten Kavität andererseits aufrechterhalten bleibt. Im übrigen ist ein erfindungsgemäß ausgebildeter Schaft 10 in Seitenansicht (ventral bzw. dorsal) dargestellt, und zwar im Vergleich zu einem herkömmlichen blattartigen Schaft entsprechend Fig. 6 bzw. EP 0 240 815 B1. Aus Fig. 1 ist sehr gut erkennbar, welche Maßnahmen im Vergleich zum Stand der Technik getroffen worden sind. Die laterale Seite des Trochanterflügels 9 gemäß Fig. 6 ist durch den lateralproximalen Bogenabschnitt 11 gekappt mit dem Vorteil, daß der Eingriff in das Trochantermassiv entsprechend geringer ausfällt und insbesondere auch Muskel- und Sehnenansätze bei der Implantation bzw. bei der Ausbildung der Kavität für den Schaft 10 weniger stark beeinträchtigt werden. Die Kontaktzonen im Bereich des sogenannten Adam'schen Bogens (Bereich 14 in Fig. 2) und am lateral-distalen Ende (Bereich 13 in Fig. 2) beschreiben einen Bogen entlang der lateralen Schulter. Dieser Bogen wird durch ein Polynom (Kurve x-ter Ordnung) beschrieben. Diese Kurve kann sich kontinuierlich an die lateral-distale Schaftgeometrie anschließen; vorzugsweise bildet sie aber mit dieser eine Verschneidung. Auf jeden Fall ist der lateral-proximale Bogenabschnitt so gestaltet, daß über den gesamten Einführweg des distalen geraden Schaftanteils die Schulter exakt Kontakt mit der oder einen konstanten Abstand zur Knochenstruktur im Trochanterbereich aufweist. Damit wird eine optimale, exakt passende Schulterpartie zum knöchernen Bett mit spaltfreiem Sitz oder exakt vorbestimmtem Spalt für Zement erzielt, je nachdem, ob zementfrei oder mit Zement implantiert werden soll.

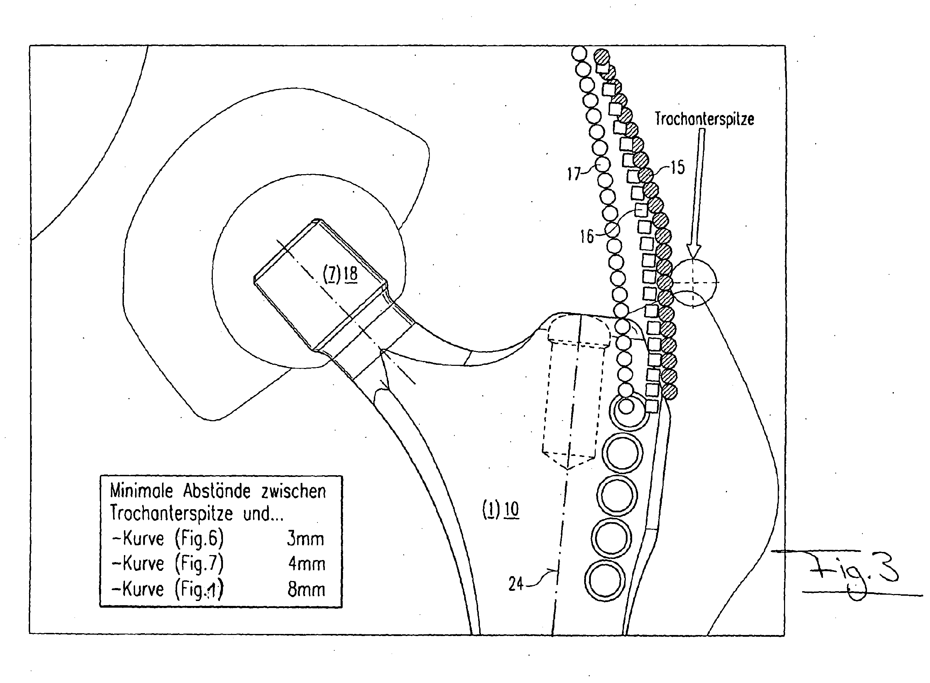

Grundsätzlich wäre es auch denkbar, den lateral-distalen Geradabschnitt im proximalen Bereich fortzusetzen entsprechend Fig. 7. Diese Ausführungsform ist jedoch bei weitem nicht so muskel- und sehnenschonend wie die erfindungsgemäße Ausführungsform gemäß Fig. 1. Dies wird insbesondere aus Fig. 3 sehr deutlich, in der die Einführwege des proximalen Endes der lateralen Schmalseite des Schaftes für die Versionen gemäß Fig. 6, Fig. 7 und Fig. 1 dargestellt sind. Der Einführweg 15 gilt für die Ausführungsform gemäß Fig. 6 (Stand der Technik). Der Einführweg 16 gilt für die Ausführungsform gemäß Fig. 7, und der Einführweg 17 gilt für die crfindungsgemäße Ausführung gemäß Fig. 1. Dementsprechend ist der Eingriff in den Trochanter bei der crfiudungsgcmäßen Ausführungsform am getingsten.

Nochmals unter Bezugnahme auf Fig. 1 sei darauf hingewiesen, daß diese einen erfindungsgemäß ausgebildeten blattartigen Schaft 10 einer Hüftgelenkprothese für die Verankerung im Femur zeigt. Dieser Schaft meist einen einen Prothesenhals 18 umfassenden Abschnitt 19 seinerseits und einen sich zu einem distalen Ende 20 hin verjüngenden Pemvx-Vexanlrexungsabschnitt 21 andererseits auf, dessen laterale Schmalseite 22 einen distalen Geradabschnitt 23 und einen prosimalen Bogeabschnitt 11 umfaßt, wobei der Geradabschnitt 23 sich über eine Länge L_{D} von 60 % bis 75 % der Gesamtlänge I_{G} des Schaftes 10 erstreckt. Der laterale Geradabschnitt 23 kann in den lateralen Bogenabschnitt 11 bei der dargestellten Ausführungsform sprungfrei übergehen, das heißt er ist tangential. Es ist jedoch - wie bereits erwähnt - durchaus sinnvoll, daß dieser Übergang einen Sprung umfaßt, das heißt stumpf-winkelig ist.

Wie bereits oben erwähnt, ist es besonders vorteilhaft, wenn der laterale Bogenabschnitt 11 als eine Art "Schleppkurve" ausgebildet ist, die das proximale Ende der lateralen Schmalseite des Schaftes 10 bei Einführung desselben in eine komplementäre Kavität im Femur unter Aufrechrerhaltung des Kontaktes zwischen lateral-distaler und proximalmedialer Schaftkontur einerseits und zugeordneter Begrenzung der Kavität andererseits beschreibt bzw. definiert. Es wird diesbezüglich nochmals auf Fig. 2 verwiesen.

Abhängig von der Größe der Schaftes sowie der äußeren Bedingungen ist der proximale Bogenabschnitt 11 vorzugsweise mit einem konstanten, insbesondere sich aber stetig oder diskret verändernden Radius von zwischen 200 mm und 500 mm ausgeführt.

Auch hat es sich als praxisnah erwiesen, wenn der proximale Bogenabschnitt 11 mit einem von distal nach proximal stetig oder diskret zunehmend größer werdenden Radius ausgeführt ist.

Der Bogenabschnitt 11 kann vorzugsweise auch als Fiyperbel-, Parabel- oder Ellipsenabschnitt ausgebildet sein dergestalt, daß der entsprechende Abschnitt nach distal in den konischen Geradabschnitt 23 in einem vorgegebenen Punkt einmündet, in dem die Tangente mit der Schaftachse 24 einen Winkel gleich dem halben Konuswinkel bildet.

Zum Schaft selbst sei noch erwähnt, daß sich der Verankerungsabschnitt über die Länge des lateral-distalen Geradabschnitts 23 vom distalen Ende 20 ausgehend in Richtung seiner Längsachse 24 entweder allseitig, oder nur lateral-medial konisch erweitert.

Des weiteren sei erwähnt, daß der Querschnitt des Schaftes 10 vorzugsweise rechteckförmig ist, aber auch trapez- oder rautenförmig sein kann.

Die erwähnte allseitig konische Erweiterung des Verankerungsabschnitts 21 weist einen Konuswinkel zwischen etwa 0,5° und 6°, insbesondere etwa 1° und 3° auf, und zwar insbesondere auch an der ventralen und/oder dorsalen Seite.

Sämtliche in den Anmeldungsunterlagen offenbarten Merkmale werden als erfindungswesentlich beansprucht, soweit sie einzeln oder in Kombination gegenüber dem Stand der Technik neu sind.

### Bezugszeichen

### Stand der Technik:

- 1: Schaft
- 2: Femur-Verankerungsabschnitt
- 3: distales Ende
- 4: Längsachse
- 5: mediale Schmalseite
- 6: Prothesenhalsachse
- 7: Prothesenhals
- 8: laterale Schmalseite
- 9: Trochanterflügel

### Erfindung:

- 10: Schaft
- 11: proximal-laterale Schaftrundung (lateral-proximaler Bogenabschnitt)
- 12: Kurve
- 13: lateral-distale Schaftkontur
- 14: proximal-mediale Schaftkontur
- 15: Einführungskurve für Schaft gemäß Fig. 6
- 16: Einführungskurve für Schaft gemäß Fig. 7
- 17: Einführungskurve für Schaft gemäß Fig. 1
- 18: Prothesenhals
- 19: Abschnitt
- 20: distales Ende
- 21: Femur-Verankerungsabschnitt
- 22: laterale Schmalseite
- 23: distal-lateraler Geradabschnitt
- 24: Schaftachse

## Patentansprüche

1. Blattartiger Schaft (10) einer Hüftgelenkprothese für die Verankerung im Femur, mit einem einen Prothesenhals (18) umfassenden Abschnitt (19) einerseits und einem sich zu einem distalen Ende (20) hin verjüngenden Femur-Verankerungsabschnitt (21) andererseits, dessen laterale Schmalseite (22) einen distalen Geradabschnitt (23) und einen proximalen Bogenabschnitt (11) umfaßt, wobei der Geradabschnitt (23) sich über eine Länge (L_{D}) von 60 % bis 75 % der Gesamtlänge (L_{G}) des Schaftes (10) erstreckt,
**dadurch gekennzeichnet, daß**
der laterale Bogenabschnitt (11) als "Schleppkurve" ausgebildet ist, die der Kurve entspricht, die das proximale Ende der lateralen Schmalseite des Schaftes (10) bei Einführung desselben, der einer entsprechenden Raspel, in eine komplementäre Kavität im Femur unter Aufrechterhaltung des Kontaktes zwischen lateral-distaler (13) und proximal-medialer (14) Schaftkontur einerseits und zugeordneter Begrenzung der Kavität andererseits beschreibt bzw. definiert.

2. Schaft nach Anspruch 1,
**dadurch gekennzeichnet, daß**
der laterale Geradabschnitt (23) in den lateralen Bogenabschnitt (11) entweder sprungfrei, d.h. tangential, oder mit Sprung, d.h. unter Winkelabweichung, übergeht.

3. Schaft nach einem der Ansprüche 1 bis 2,
**dadurch gekennzeichnet, daß**
der proximale Bogenabschnitt (11) mit einem konstanten, insbesondere sich aber stetig oder diskret verändernden Radius von zwischen 200 mm und 500 mm ausgeführt ist.

4. Schaft nach einem der Ansprüche 1 bis 3,
**dadurch gekennzeichnet, daß**
der proximale Bogenabschnitt (11) mit einem von distal nach proximal stetig oder diskret zunehmend größer werdenden Radius ausgeführt ist.

5. Schaft nach einem der Ansprüche 1 bis 4,
**dadurch gekennzeichnet, daß**
sich der Verankerungsabschnitt über die Länge des lateral-distalen Geradabschnitts (23) vom distalen Ende (20) ausgehend in Richtung seiner Längsachse (24) entweder allseitig, oder nur lateral-medial konisch erweitert.

6. Schaft nach einem der Ansprüche 1 bis 5,
**dadurch gekennzeichnet, daß**
sein Querschnitt rechteck-, trapez- oder rautenförmig ist.

7. Schaft nach Anspruch 5 oder 6,
**dadurch gekennzeichnet, daß**
bei allseitig konischer Erweiterung des Verankerungsabschnitts (21) der Konuswinkel, insbesondere an der ventralen und/oder dorsalen Seite zwischen etwa 0,5° und 6°, insbesondere etwa 1° und 3° beträgt.

## Claims

1. Blade-like stem (10) of a hip joint prosthesis for anchoring in the femur, having a portion (19) comprising a prosthesis neck (18) on the one hand and a femur-anchoring portion (21) tapering towards a distal end (20) on the other hand, the lateral narrow side (22) of which comprises a distal straight portion (23) and a proximal arcuate portion (11), the straight portion (23) extending over a length (L_{D}) of from 60 % to 75 % of the total length (L_{G}) of the stem (10),
**characterized in that**
the lateral arcuate portion (11) is in the form of a "tractrix" which corresponds to the curve described or defined by the proximal end of the lateral narrow side of the stem (10) on introduction thereof, or of a corresponding rasp, into a complementary cavity in the femur while the contact between the lateral-distal (13) and proximal-medial (14) stem contour on the one hand and the associated boundary of the cavity on the other hand is maintained.

2. Stem according to claim 1,
**characterized in that**
the lateral straight portion (23) merges into the lateral arcuate portion (11) either continuously, that is to say tangentially, or with a discontinuity, that is to say with angular deviation.

3. Stem according to either one of claims 1 and 2,
**characterized in that**
the proximal arcuate portion (11) is configured with a constant, however especially continuously or discontinuously changing radius of between 200 mm and 500 mm.

4. Stem according to any one of claims 1 to 3,
**characterized in that**
the proximal arcuate portion (11) is configured with a radius that becomes increasingly larger continuously or discontinuously from distal to proximal.

5. Stem according to any one of claims 1 to 4,
**characterized in that**
the anchoring portion widens conically over the length of the lateral-distal straight portion (23) starting from the distal end (20) in the direction of its longitudinal axis (24) either all round or only laterally-medially.

6. Stem according to any one of claims 1 to 5,
**characterized in that**
its cross-section is rectangular, trapezoidal or rhombic.

7. Stem according to claim 5 or 6,
**characterized in that**
in the case of an anchoring portion (21) that widens conically all round, the cone angle is about from 0.5° to 6 °, especially about from 1° to 3°, particularly on the ventral and/or dorsal side.

## Revendications

1. Tige en forme de lame (10) d'une prothèse de hanche pour l'ancrage dans le fémur, avec une section (19) comprenant un col de prothèse (18) d'un côté et une section d'ancrage dans le fémur (21) s'effilant en direction de son extrémité distale (20) de l'autre côté, dont le côté latéral étroit (22) comprend une section distale droite (23) et une section proximale arquée (11), la section droite (23) s'étendant sur une longueur (L_{D}) de 60 % à 75 % de la longueur totale (L_{G}) de la tige (10),
**caractérisée en ce que**
la section latérale arquée (11) est réalisée comme tractrice qui correspond à la courbe que décrit, respectivement définit l'extrémité proximale du côté latéral étroit de la tige (10) lors de l'introduction de celle-ci, ou d'une râpe correspondante, dans une cavité complémentaire dans le fémur moyennant la conservation du contact entre les contours lateral-distal (13) et proximal-médial (14) de la tige d'un côté et la limitation correspondante de la cavité de l'autre côté.

2. Tige selon la revendication 1,
**caractérisée en ce que**
la section latérale droite (23) se prolonge dans la section latérale arquée (11) soit sans décrochement, c'est-à-dire tangentiellement, soit avec décrochement, c'est-à-dire moyennant un écart angulaire.

3. Tige selon l'une quelconque des revendications 1 à 2,
**caractérisée en ce que**
la section proximale arquée (11) est exécutée avec un rayon constant, mais en particulier variant continûment ou discrètement entre 200 mm et 500 mm.

4. Tige selon l'une quelconque des revendications 1 à 3,
**caractérisée en ce que**
la section proximale arquée (11) est exécutée avec un rayon augmentant continûment ou discrètement du côté distal au côté proximal.

5. Tige selon l'une quelconque des revendications 1 à 4,
**caractérisée en ce que**
la section d'ancrage s'élargit coniquement sur la longueur de la section latérale-distale droite (23), en partant de l'extrémité distale (20) dans la direction de son axe longitudinal (24), soit dans toutes les directions, soit seulement dans la direction latérale-médiale.

6. Tige selon l'une quelconque des revendications 1 à 5,
**caractérisée en ce que**
sa section est rectangulaire, trapézoïdale ou rhomboédrique.

7. Tige selon la revendication 5 ou 6,
**caractérisée en ce que**
pour un élargissement conique de la section d'ancrage (21) dans toutes les directions, l'angle du cône, en particulier sur la face ventrale et/ou dorsale, est compris environ entre 0,5° et 6°, en particulier entre 1° et 3°.
